# EUROPEAN PATENT SPECIFICATION

(11) **EP 1 294 864 B1**
(45) Date of publication and mention of the grant of the patent: **15.03.2006**
(21) Application number: 01956445.9
(22) Date of filing: 25.05.2001
(51) Int. Cl.: C12N 9/64

(54) **MUTATED FURIN POLYPEPTIDES HAVING IMPROVED CHARACTERISTICS**
MUTIERTE FURIN POLYPEPTIDE, WELCHE VERBESSERTE EIGENSCHAFTEN AUFWEISEN
POLYPEPTIDES FURINE AUX CARACTERISTIQUES AMELIOREES

(30) Priority: 09.06.2000 US 592480
(43) Date of publication of application: 26.03.2003
(73) Proprietor: Baxter Aktiengesellschaft, 1221 Wien (AT)
(72) Inventor: PLAIMAUER, Barbara, A-1020 Vienna (AT); SCHLOKAT, Uwe, A-2304 Orth/Donau (AT)
(74) Representative: Müller-Boré & Partner Patentanwälte
(86) International application number: PCT/EP2001/005991
(87) International publication number: WO 2001/094383

(56) References cited:
- US-A- 5 460 950
- PREININGER ALEXANDRA ET AL: "Strategies for recombinant Furin employment in a biotechnological process: Complete target protein precursor cleavage." CYTOTECHNOLOGY, vol. 30, no. 1-3, 1999, pages 1-15, XP001094272 ISSN: 0920-9069
- DATABASE EMBL [Online] 1 November 1996 (1996-11-01) SPENCE MJ, ET AL.: "Furin endoprotease" Database accession no. Q60426 XP002207527
- DATABASE EMBL [Online] 8 May 1998 (1998-05-08) HOECHST: "Human BMP processing enzyme furin" Database accession no. AAW36099 XP002207528 & WO 97 41250 A (HOECHST) 6 November 1997 (1997-11-06)
- SMEEKENS S P ET AL: "IDENTIFICATION OF A CDNA ENCODING A SECOND PUTATIVE PROHORMONE CONVERTASE RELATED TO PC2 IN ATT20 CELLS AND ISLETS OF LANGERHANS" PROCEEDINGS OF THE NATIONAL ACADEMY OF SCIENCES OF USA, NATIONAL ACADEMY OF SCIENCE. WASHINGTON, US, vol. 88, 1991, pages 340-344, XP002023317 ISSN: 0027-8424
- PLAIMAUER BARBARA ET AL: "'Shed' furin: Mapping of the cleavage determinants and identification of its C-terminus." BIOCHEMICAL JOURNAL, vol. 354, no. 3, 2001, pages 689-695, XP002207526 ISSN: 0264-6021

## Description

### Background of the Invention

The present invention relates to new furin polypeptides. Furin, also called PACE (for paired basic amino acid cleavage enzyme), belongs to the family of mammalian subtilisin-like proprotein convertases (SPC or PC). These proteins have been implicated in the endoproteolytic maturation processing of inactive precursor proteins at single, paired or multiple basic consensus sites within the secretory pathway (reviewed in Nakayama, 1997, Biochem.J., 327, pp. 625-635; (Seidah and Chretien, Current Opinions in Biotechnology,8, 1997, pp. 602-607). Seven distinct members of this family have been identified to date, including furin, PC1 (also known as PC3), PC2, PACE4, PC4, PC5 (also known as PC6), PC7 (or LPC, PC8, or SPC7), each of which exhibits unique tissue distribution, although overlapping functional redundancy of various PCs in some tissues may occur (Seidah et al., Biochem.,1994, 76, pp. 197-209).

Furin is ubiquitously expressed in all mammalian tissues and cell lines which have been examined, and is capable of processing a wide range of bioactive precursor proteins in the secretory pathway, including growth factors, hormones, plasma proteins, receptors, viral envelope glycoproteins and bacterial toxins. It is a calcium-dependent serine endoprotease structurally arranged into several domains, namely a signal peptide, propeptide, catalytic domain, middle domain, (also termed homo-B or P-domain), the C-terminally located cysteine-rich domain, transmembrane domain and the cytoplasmic tail. Upon transit of the newly synthesized furin precursor from the endoplasmic reticulum to the Golgi compartment, the propeptide is autocatalytically removed in a two step processing event (Leduc et al., J.Biol.Chem., 267, 1992, pp. 14304-14308; Anderson et al., EMBO J., 1997, pp. 1508-1518).

Furin is predominantly localized to the trans-Golgi network (TGN), but it also cycles between the TGN and the cell surface via endosomal vesicles, thereby processing both precursor proteins during their transport through the constitutive secretory pathway as well as molecules entering the endocytic pathway. The cellular distribution of furin to the varied processing compartments is apparently directed by defined structural features within its cytoplasmic tail (Schafer et al., EMBO J.,11, 1995, pp. 2424-2435; Voorhees et al., EMBO J., 20, 1995, pp. 4961-4975; Teuchert et al., J.Biol.Chem., 274, 1999, pp. 8199-8207). Deletion of the cytoplasmic domain results in a truncated furin polypeptide located primarily in the plasma membrane, to which it is transported probably by a default pathway, incapable of recycling to the TGN due to the loss of regulative sequence motifs within the cytoplasmic domain (Molloy et al., EMBO J., 13, 1994, pp. 18-33; Schafer et al., EMBO J., 14, 1995, pp. 2424-2435).

The C-terminal domains have been found to be dispensable for the functional activity of furin. Mutant furin lacking the transmembrane domain and the cytoplasmic tail, was found to be readily released into cell culture medium while still exhibiting significant activity. High levels of expression of full length recombinant furin have resulted in the natural secretion of a truncated furin form, called 'shed' furin, which lacks the transmembrane domain and the cytoplasmic tail (Wise et al., Proc.Natl.Acad.Sci., 87, 1990, pp. 9378-9382; Rehemtulla and Kaufman, Blood, 79, 1992, pp. 2349-2355; Vidricaire et al., Biochem.Biophys.Res.Comm., 195, 1993 pp. 1011-1018; Vey et al., J.Cell.Biol., 127, 1994, pp. 1829-1842; Preininger et al.,Cytotechnol., 30, 1999, pp. 1-15). It remains an open question as to whether furin shedding is due to saturating cellular retrieval mechanisms, whether it represents a protection mechanism of the host cell against excess protease, or whether is part of a natural regulatory process modulating intracellular furin concentration/activity by secretion. The isolation of a truncated endogenous furin from the Golgi fraction of bovine kidney cells may support the view that shedding is not solely an artificial secretion process caused by overexpression (Vey et al., 1994). Conversion of furin into the soluble secreted form was shown to occur intracellularly within an acidic compartment which requires the presence of calcium (Vey et al., 1994) .

The presence of a C-terminal truncated and hence soluble form of furin that remains active, however, has been detected almost exclusively in conditioned medium of cells recombinantly overexpressing native full-length furin (Wise et al., 1990; Rehemtulla and Kaufman, 1992; Vidricaire et al., 1993; Vey et al., 1994; Preininger et al., 1999).

Other prior art describing furin polypeptides includes WO 91/06314, which describes a fragment of furin consisting of amino acids 108-464, thus lacking part of the homo-B domain, the cysteine-rich region, the transmembrane domain and the cytoplasmic tail. WO 92/09698 discloses full length furin and furin lacking the transmembrane domain. In addition, Preininger et al. (Cytotechnology 30, 1999, pp. 1-15) describe furin mutants lacking the cysteine rich region, the trans-membrane domain and the cytosolic domain. Cells expressing such mutants contained increased intracellular concentrations of the furin derivatives but varying levels of secretion. The authors stated that the lack of extracellular accumulation of these molecules suggested that these molecules were most likely degraded. The authors stated further that full length recombinant furin, located intracellularly, seems to be largely inactive and that there is a potential toxicity of larger amounts of full length furin to its host cell.

### Summary of the Invention

We have found that soluble furin in a cell culture medium can cause proteins which are not naturally processed by furin to be unspecifically cleaved. For example, although native Factor VIII is not naturally processed by furin, Factor VIII can become a target for inadvertant processing by -soluble furin when exposed to furin for an extended period of time, e.g. in a cell culture medium. This leads to a reduced yield of structurally intact Factor VIII protein in such cell culture medium. This can be the case when Factor VIII is coexpressed together with a natural substrate of furin, e.g. von Willebrand Factor,or when recombinant proteins which are naturally processed by furin are exposed to furin for an extended period of time so that in addition inadvertent sites are cleaved.

The present invention reduces or prevents unspecific cleavage of proteins in cell culture through the use of modified furin polypeptides which have proteolytic activity but which are not secreted into culture medium by host cells or are secreted in reduced amounts compared to the secretion of wild-type furin. Such furin polypeptides have been found not to be toxic to host cells even when expressed intracellularly in high amounts.

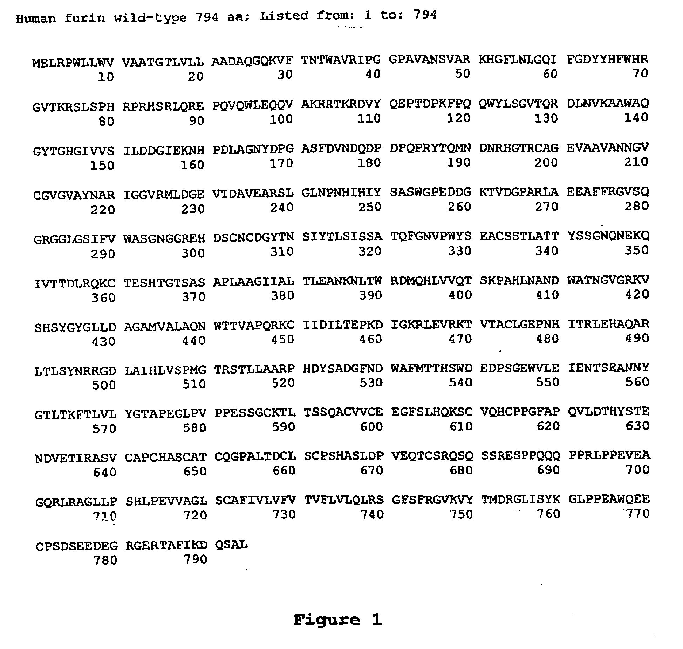

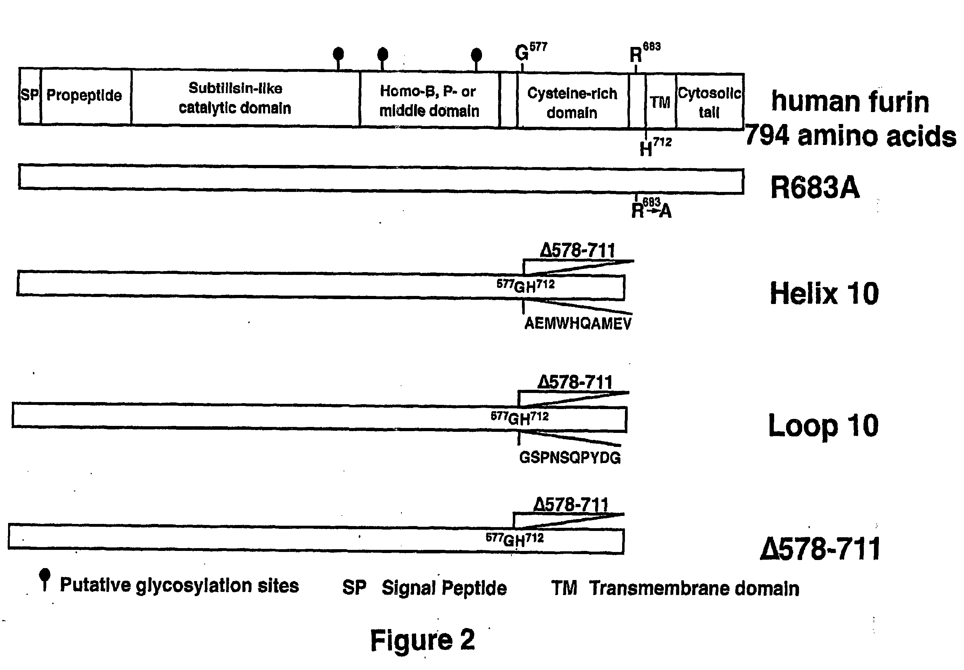

Accordingly, the present invention provides a furin polypeptide having a modified amino acid sequence compared to that of wild-type furin between homo-B-domain and the transmembrane domain, that is, between amino acids Ala 557 and Leu 713 according to the amino acid sequence presented in Figures 1 and 2. It has been surprisingly found that furin polypeptides having such a modified amino acid sequence have proteolytic activity similar to that of native (i.e., wild-type) furin, but are secreted by host cells expressing such furin polypeptides into cultivation medium in substantially reduced amounts compared to native furin.

It is another aspect of the invention that the furin polypeptides according to the invention can be expressed in high amounts in a cell without being substantially toxic to the cell. In still a further aspect, the physiological cleavage properties of the modified furin protein are still present, but inadvertent cleavage of secreted or extracellularly localized proteins in a cell culture medium is highly reduced since less or no furin is present in the medium.

Additionally, a further advantage of the furin polypeptide of the present invention is that although the proteolytic processing of furin-dependent proteins can occur intracellularly, unspecific processing of proteins by furin can be at least reduced if not completely eliminated. Therefore, unspecific cleavage of proteins which might occur when proteins are exposed to soluble furin in a conditioned medium in cell culture is avoided by the furin polypeptide according to the present invention.
In another aspect, the invention provides a recombinant polynucleotide encoding the furin polypeptide according to the present invention. In yet another aspect, the invention provides a method for producing the furin polypeptide according to the present invention, a recombinant vector comprising the polynucleotide sequence encoding the furin polypeptide according to the invention, a host cell comprising such vector, and a preparation comprising the furin polypeptide of the present invention.

### Description of the Drawings

Figure 1 shows the amino acid sequence of human wild-type furin.
Figure 2 is a schematic representation of the amino acid sequences of wild-type furin and furin mutants.
Figure 3 is a photograph of an SDS-PAGE gel showing shed furin in a conditioned medium in which FD11-CHO-rvWF cells transiently transfected with furin constructs were grown.
Figure 4 is a photograph of an SDS-PAGE gel showing the processing of rvWF precursor in FD11-CHO-rvWF cells transiently transfected with furin constructs.
Figures 5A-5C show furin expression in transiently transfected HEK 293 cells:

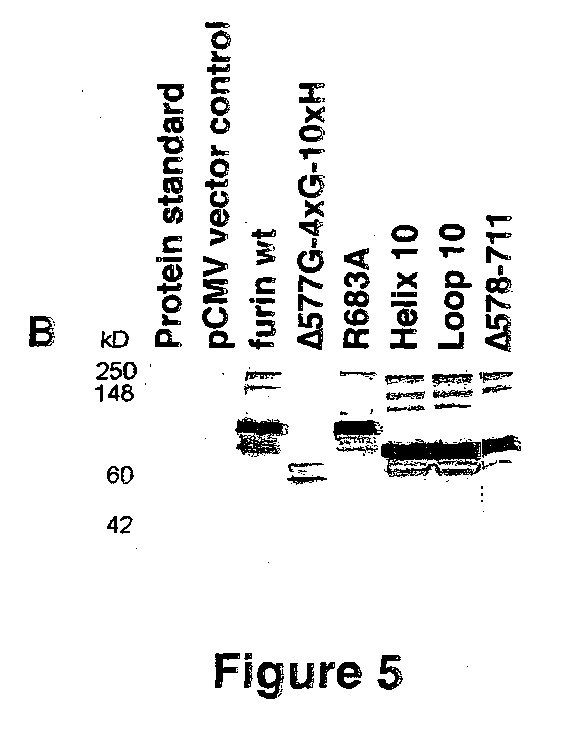

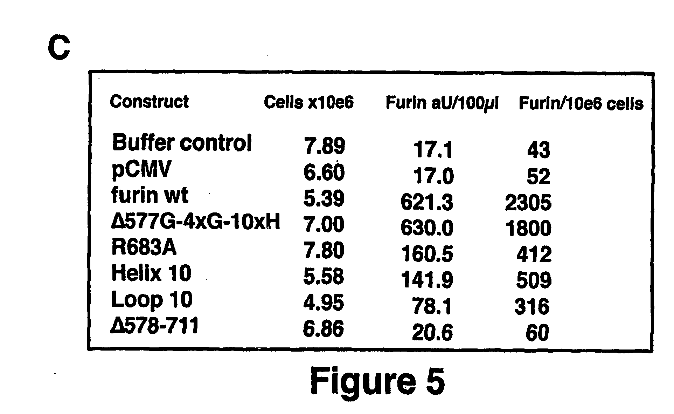

   Figure 5A is a photograph of an SDS-PAGE gel showing shed recombinant furin (rfurin) in conditioned medium of transiently transfected HEK 293 cells;
   Figure 5B is a photograph of an SDS-PAGE gel showing intracellular rfurin expression in HEK 293 lysates; and
   Figure 5C shows the results of an in vitro furin assay using conditioned medium and fluorogenic substrate (in arbitrary units).

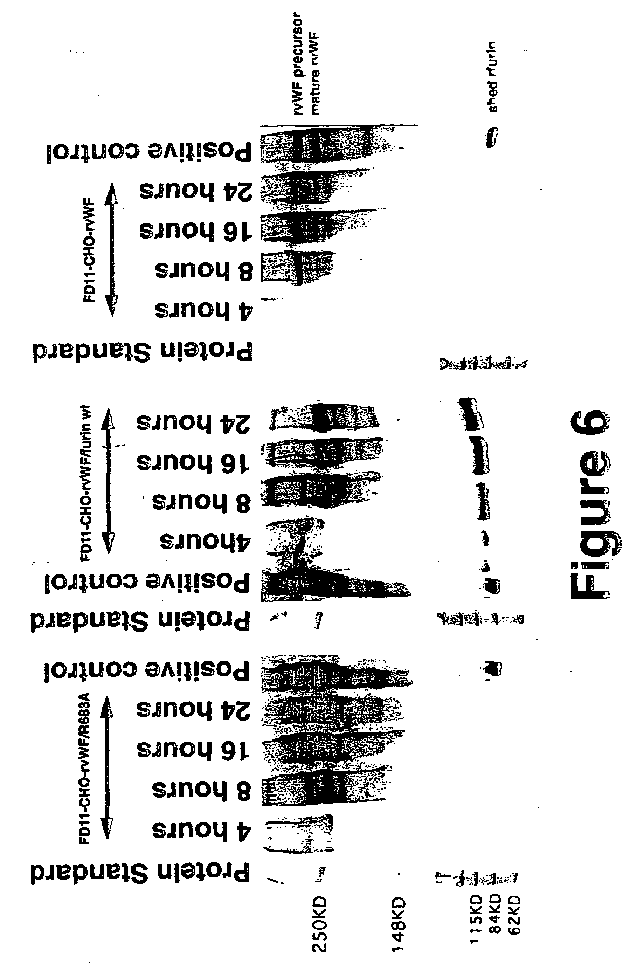

Figure 6 is a photograph of three SDS-PAGE gels showing the correlation between the degree of rvWF precursor processing and the presence of shed furin in conditioned medium.

### Detailed Description of the Invention

### Furin Polypeptides

The present invention comprises furin polypeptides which have a modified amino acid sequence between amino acids Ala 557 and Leu 713 compared to the amino acid sequence of wild-type mammalian furin, such as human furin (the amino acid sequence of which is shown in Figure 1). For purposes of the present disclosure, a furin polypeptide shall refer to a polypeptide comprising at least a portion of the amino acid sequence of a wild-type mammalian furin protein which has proteolytic activity. In a preferred embodiment the modification in a furin polypeptide according to the present invention is located between amino acids Ala 557 and Leu 713. In an alternative embodiment, the modification is at Arg 683. In still another embodiment, the amino acids between Gly 577 and His712 are deleted.

In the instant disclosure, the terms "modified" and "modification" shall mean, with respect to the amino acid sequence of a furin polypeptide, an addition, deletion or substitution of one or more amino acids. Such a modification can be carried out by, for instance, directed mutagenesis or PCR or other methods of genetic engineering known in the art which are suitable for specifically changing a DNA sequence in order to direct a change in the amino acid sequence of the resulting polypeptide (Current Protocols in Molecular Biology, vol. 1, ch. 8 (Ausubel et al. eds., J. Wiley and Sons, 1989 & Supp. 1990-93); Protein Engineering (Oxender & Fox eds., A. Liss, Inc., 1987). The modifications of the present invention are in the region between the homo-B-domain and the transmembrane domain, i.e. the region between the amino acids Ala 557 and Leu 713, of the furin molecule.

Preferably, the furin polypeptide of the present invention has amino acid substitutions and/or additions creating loop or alpha-helix structures. It is well known from the prior art that amino acids can form several different secondary structures in polypeptides, i.e. helical or looped structures (Lehninger A., "Biochemie", VCH, 1985, pp. 102-107; Karlson P. et al., "Kurzes Lehrbuch der Biochemie, Georg Thieme Verlag, 1994; pp. 29-32). These structures can be produced by selecting specific amino acids which form, for example, alpha helices and loops and thereby developing structures like helices or loops in the resulting polypeptide (Rost B. and Sander C., Proc.Natl.Acad.Sci., 1993, pp. 7558-7562, Rost B. and Sander C., 1994, Proteins: Structure, Function and Genetics, 19, pp. 55-72). Additionally, according to Kyte J. and Doolittle R. (1983, J.Mol.Biol., 157, pp. 105-132) such amino acids may be selected based on their hydropathy values, in view of the knowledge that amino acids showing negative hydropathy values are hydrophilic, allowing these side chains access to the aqueous solvent, whereas amino acids showing positive hydropathy values are hydrophobic amino acids which tend to comprise interior portions of the proteins. Additionally, it is known that amino acids showing very high positive or negative hydropathy values are preferred targets for various proteases.

Therefore, in a preferred embodiment, there is an insertion of several amino acids, preferably between 5 and 30, more preferably between 10 to 20, which produce a loop or helix structure in the modified furin polypeptide of the present invention.

In an alternative embodiment, the insertion of amino acids results in a helix structure. In such an embodiment the amino acids are preferably selected from the group consisting of alanine (A), leucine (L), phenylalanine (F), tryptophan (W), methionine (M), histidine (H), glutamine (Q), valine (V) and glutamic acid (E). For example, the amino acids 558 to 738, preferably amino acids 578 to 711 are substituted by the amino acid sequences EAMHA, (SEQ.ID.No 1) AWFQW (SEQ.ID.No 2) OR AQMWHEAMEFWAMQFEAMHA (SEQ.ID.No 3). In a preferred embodiment, amino acids 578 to 711 of the furin polypeptide are substituted by the amino acid sequence AEMWHQAMEV (SEQ.ID.No 4).
In yet another embodiment, an amino acid insertion builds up a loop structure, wherein the amino acids are preferably selected from the group consisting of serine (S), isoleucine (I), threonine (T), glutamic acid (E), aspartic acid (D), lysine (K), arginine(R), glycine (G), tyrosine (Y), cysteine(C), asparagine (N), proline (P), glutamine (Q) and hydroxyproline. For example, the amino acids 558 to 738, preferably amino acids 578 to 711 are substituted by the amino acid sequences SYNPG, SYQPD or GSPYQTNGPS. In a preferred embodiment, amino acids 578 to 711 of the furin polypeptide are substituted by the amino acid sequence GSPNSQPYDG (SEQ. ID. No 5).

The selection of amino acids for forming looped and helical structures is well known to the skilled person (Lehninger A., "Biochemie", VCH, 1985, pp. 102-107).

In an alternative embodiment, the arginine at amino acid position 683 of the furin sequence can be replaced by any of the amino acids, preferably by lysine, glutamic acid or isoleucine.

### Nucleic Acids and Vectors

Another embodiment of the invention provides polynucleotides which encode the furin polypeptides of the present invention. The nucleic acids used in such polynucleotides may be DNA and/or RNA.

A full-length furin polynucleotide as well as any derivatives thereof encoding a furin polypeptide having proteolytic activity can be used as the starting material for the construction of the furin polypeptides of the present invention. The cDNA sequence encoding native human furin was published by van den Ouweland, A.M.W. et al. (Nucleic Acid Res., 1990, 18(3), p. 664) and Fuller R.S. et al. (Science, 1989, 246:482). Such a furin polynucleotide can originate from any mammalian species, preferably from human, porcine or bovine sources.

The polynucleotide is expressed by a vector that provides the appropriate elements for the heterologous expression of said DNA or RNA. The expression vector comprises, for example, a transcriptional regulatory region and a translational initiation region functional in a host cell, a DNA sequence encoding for the furin polynucleotide of the present invention and translational and transcriptional termination regions functional in said host cell, wherein expression of said nucleic sequence is regulated by said initiation and termination regions.

The expression vector may also contain elements for the replication of said DNA or RNA. The expression vector may be a DNA or an RNA vector. Examples for DNA cloning and expression vectors are pBSSKII (Short, J.M., Fernandez, J.M., Sorge, J.A. and Huse, W.D. Lambda ZAP, 1988, Nucleic Acids Research 16 (15), 7583-7600; Alting. Mees, M.A., and Short, J.M., 1989, Nucleic Acids Research 17 (22), 9494), pBPV, pSVL, pCMV, pRc/RSV, myogenic vector systems (WO 93/09236) or vectors derived from viral systems, for example from vaccinia virus, adenoviruses, adeno-associated virus, herpesviruses, retroviruses or baculoviruses. Examples for RNA expression vectors are vectors derived from RNA viruses like retroviruses or flaviviruses.

In some instances it might be desirable to have a plurality of copies of the gene expressing the protein precursor in relation to the furin polypeptide, or vice versa. This can be achieved in ways well described in the prior art. Alternatively, one can employ two transcriptional regulatory regions having different rates of transcriptional initiation or different promoters, providing for enhanced expression of either the furin polypeptide according to the invention or the expression of the precursor polypeptide and/or a further polypeptide which is not to be proteolytically processed by furin.

The expression vector containing the polynucleotide which encodes the modified furin polypeptide according to the present invention can be used to transform host cells which then produce said polypeptide. The transformed host cells can be grown in a cell culture system to produce said polypeptide *in vitro.*

For some specific applications in gene therapy, i. e. when the nucleic acid per se is injected into an organ of a mammal, the nucleic acid, DNA as well as RNA, may be chemically modified. The chemical modifications may be modifications that protect the nucleic acid from nuclease digestion, for example by stabilizing the backbone or the termini.
The expression vector containing the nucleic acid which encodes a furin polypeptide of the present invention can further be administered to a mammal without prior in vitro transformation into host cells. The practical background for this type of gene therapy is disclosed in several patent applications, for example in WO 90/11092. The expression vector containing said nucleic acid is mixed with an appropriate carrier, for example a physiological buffer solution, and is injected into an organ, preferably skeletal muscle, the skin or the liver of a mammal.

### Host Cells

The modified furin polypeptide according to the present invention is preferably produced by recombinant expression. It can be prepared by means of genetic engineering with expression systems known to the art, such as, for instance, permanent cell lines or viral expression systems. Permanent cell lines are prepared by stable integration of the extraneous DNA into the host cell genome of, e.g., vero, MRCS, CHO, BHK, 293, HEK 293, Sk-Hep1, liver cells, kidney cells, fibroblasts, keratinocytes or myoblasts, hepatocytes or stem cells, for example hematopoietic stem cells, or by an episomal vector derived, for example, from papilloma virus.

Alternatively, cell lines having no endogenous furin activity can be used (Moehring J.M. and Moehring T.J., Infect.Immun., 41, 1983, pp. 998-1009). For example, CHO-RPE40 or FD11-CHO-cells can be used. Therein, the proteolytic activity of the transfected furin of the invention can be easily measured, avoiding the background activity of endogenous furin.

Viral expression systems, such as, for instance, the vaccinia virus, baculovirus or retroviral systems, can also be employed. As cell lines, vero, MRC5, CHO, BHK, 293, Sk-Hep1, gland, liver or kidney cells are generally used. Eukaryotic expression systems, such as yeasts, endogenous glands (e.g. glands of transgenic animals) and transgenic animals can also be used for the expression of the furin polypeptides according to the present invention. For the expression of recombinant proteins, CHO-DHFR-cells have proved particularly useful (Urlaub et al., Proc.Natl.Acad.Sci., USA, vol 77, pp. 4216-4220, 1980).

The furin polypeptides according to the present invention are expressed in the respective expression systems under the control of suitable promoters. For expression in eukaryotes, known promoters are suitable, such as SV40, CMV, RSV, HSV, EBV, β-actin, hGH or inducible promoters such as hsp or metallothionein promoter.

In a preferred embodiment the present invention provides a method for the production of a furin polypeptide according to the present invention and a precursor polypeptide. Preferably, the furin polypeptide is coexpressed with von Willebrand factor protein and/or Factor VIII protein.

In a further aspect the invention provides a method for the production of a furin polypeptide according to the present invention. This method comprises growing in a nutrient medium a host cell comprising an expression vector which comprises, in the direction of transcription, a transcriptional regulatory region and a translational initiation region functional in a host cell, a DNA sequence encoding a furin polypeptide of the invention, and translational and transcriptional termination regions functional in said host cell. The expression of this DNA sequence is regulated by the initiation and termination regions. The method can further include measuring the secretion rate of expressed furin polypeptides with proteolytic activity and isolating host cells expressing furin polypeptides showing reduced secretion compared to host cells expressing wild-type furin.

### Pharmaceutical Preparation

The furin polypeptide according to the present invention can be provided as a pharmaceutical preparation having a modified furin polypeptide according to the present invention as a single component preparation or in combination with other components as a multiple component system. In a particular embodiment, a furin polypeptide of the invention can be combined with pro-proteins, for example von Willebrand Factor.

### Specific Activity

According to one aspect of the present invention, the furin polypeptide of the invention has a furin proteolytic activity of at least 50%, preferably at least 100% compared to the proteolytic activity of wild-type furin protein, such as wild-type human furin.

The evaluation of proteolytic activity can be performed by any suitable test, for example by using fluorogenic substrates which are comprised of a dibasic cleavage site for which furin is specific (Preininger A. et al., 1999, Schlokat U. et al., 1996, Biotechnol. Appl. Biochem., vol. 24, pp. 257-267). Alternatively the proteolytic activity can also be measured by incubating furin with pro-proteins, for example pro-rvWF, for a sufficient time. The degree of pro-rvWF processing can be analysed by Western blotting.

### Secretion Rate

The secretion rate can be defined as the amount of secreted furin polypeptide (shed furin) which accumulates in a cell culture medium within a given time. The reduction in the secretion rate of the modified furin polypeptide according to the present invention is at least 25%, preferably at least 50%, more preferably at least 90%, most preferably 100% compared to the secretion rate of recombinantly expressed furin having the wild-type sequence (such as wild-type human furin) or furin lacking the transmembrane and/or cytoplasmic region.

For example, the secretion rate can be measured by immunological reactivity with anti-furin antibodies. A suitable antibody can be directed against the catalytic domain of furin (Preininger et al., 1999)

### Isolation Methods

The furin polypeptide according to the present invention can be isolated from cells by lysis and further purified by conventional methods, optionally in the presence of protease inhibitors. The purification can be done by chromatographic methods known in the art, preferably by affinity chromatography, using antibodies against the furin polypeptide or by coupling the furin polypeptide to a His-Tag group and selectively binding the protein on Ni²⁺-NTA agarose (Preininger et al., 1999)

Due to the fact that the proteolytic characteristics of the furin polypeptides of the present invention compared to wild type furin are substantially unaltered, proteins that are processed by wild-type furin can also be processed by the furin polypeptides of the invention, i.e. proteins with paired amino acid residues can serve as a substrate. Examples of precursor molecules for use in the present invention can include, but are not limited to, von Willebrand Factor, Factor IX, protein C, protein S, prothrombin, Factor X, Factor VII, transforming growth factor (TGF) beta and its superfamily, including activin and inhibin, bone morphogenetic proteins (BMP), insulin, relaxin, growth factors like platelet derived growth factor (PDGF), nerve growth factor (NGF), and virus polypeptides including those from cytomegalovirus (CMV), human immunodeficiency virus and herpes simplex virus.

The invention is illustrated in the subsequently described examples. Variations within the purview of one skilled in the art are to be considered to fall within the scope of the present invention. Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. The following examples illustrate the present invention but do not limit the scope of the invention in any way.

### Examples

### 1. Construction of furin R683A.

Full length furin mutant R683A, harboring the amino acid alanine instead of the native arginine at position 683, was constructed using a PCR-based approach with overlapping extended primers (Ho et al., 1989, Gene, 77, pp. 51-59). Initially, two standard PCR reactions were performed using plasmid pCMV-furin wt (harboring the furin wild-type cDNA) as template and primer pairs 4953 (5' GGGGGATCCC TCTGGCGAGT GG 3') (SEQ.ID.No 6) and 5210 (5' CGGGGACTCT GCGCTGCTCT G 3') (SEQ.ID.No 7) or 5209 (5' CAGAGCAGCG CAGAGTCCCC G 3') (SEQ.ID.No 8) and 4954 (5' GGGGGATCCC CGCGGCCTAG G 3') (SEQ.ID.No 9), where 5210 and 5209 are the inner complementary extended primers introducing the mutation, and 4953 and 4954 are the outer primers containing a Bam HI restriction site. In a second PCR round, the two purified amplification products of the initial PCR reactions were combined for overlap extension in the presence of the two outer primers 4953 and 4954. The final purified PCR product was digested with Bam HI and was used to replace the wild-type Bam HI fragment in plasmid pCMV-furin wt.

### 2. Construction of furin deletion mutants Helix 10, Loop 10 and Δ578-711.

Furin expression constructs Helix 10 (comprising a deletion of amino acid residues 578-711 replaced by 10 helical structured residues), Loop 10 (comprising a deletion of amino acid residues 578-711 replaced by 10 loop structured residues) and Δ578-711 (comprising a deletion of amino acid residues 578-711) were generated by inverse PCR. For that purpose, the internal 1176bp Bam HI fragment of wild-type furin was subcloned into the Bam HI site of vector pBS SKII(+) (Stratagene). The resulting plasmid pBS/fur1176 was used as the template for the inverse PCR reactions of the individual constructs. In the case of Helix 10 and Loop 10, the specific sense and reverse primers each contained at their 5'-end an additional overhanging 15 nucleotides coding for 5 helical or loop structured amino acids. The following primer sets were used: for Helix 10, sense primer 5699 (5' CAGGCCATGG AGGTGCACCT GCCTGAGGTG GTGGCCGGCC TCAGC 3') (SEQ.ID.No 10) and reverse primer 5700 (5' GTGCCACATC TCGGCCCCCT CAGGGGCGGT GCCATAGAGT ACGAG 3') (SEQ.ID.No 11), for Loop 10, sense primer 5701 (5' CAGCCCTACG ACGGCCACCT GCCTGAGGTG GTGGCCGGCC TCAGC 3') (SEQ.ID.No 12) and reverse primer 5702 (5' GCTGTTGGGG CTGCCCCCCT CAGGGGCGGT GCCATAGAGT ACGAG 3') (SEQ.ID.No 13), and for Δ578-711, sense primer 5723 (5' CACCTGCCTG AGGTGGTGGC C 3') (SEQ.ID.No 14) and reverse primer 5724 (5' CCCCTCAGGG GCGGTGCCAT A 3') (SEQ.ID.No 15). The resulting PCR-fragments were purified, treated with T4 polynucleotide kinase (New England Biolabs), religated with T4 DNA-ligase (Roche) and transformed into E. coli strain XL1 Blue MRF' (Stratagene). Positive clones, harboring the introduced mutation were selected by sequencing, and the mutated BamHI fragment was used to replace the wt 1176bp BamHI fragment in pCMV-furin wt.

Generally, amplification of the target sequences was routinely carried out within 30 PCR cycles using 10-20ng template DNA in a total volume of 100µl containing 30pMol of each primer, 200µM of each dNTP, 2mM MgSO₄ in the supplied 10x PCR buffer and 2.5U Vent_{R}® DNA polymerase (New England Biolabs) at 55°C annealing and 72°C extension temperatures. PCR-fragments were purified using QIAEX II Gel Extraction Kit (Qiagen) according to the supplier's instructions.

The Helix 10 insertion into the furin deletion mutant Δ578-711 comprises the amino acid sequence AEMWHQAMEV (SEQ.ID.Nr.4).
The Loop 10 insertion into the furin deletion mutant Δ578-711 comprises the amino acid sequence GSPNSQPYDG (SEQ.ID.Nr.5) :

### 3. Transfection, cell culture and protein harvest.

Furin constructs were transiently expressed in 293 HEK (human embryonic kidney fibroblasts; ATCC CRL 1573) and FD11-CHO-rvWF cells (FD11-CHO are furin deficient cells). The cells were grown in DMEM/Ham's F12 (1:1) medium (Life Technologies) supplemented with 10% fetal calf serum (full medium). For transfection, cells were grown to 50-75% confluency on 5cm culture dishes (Costar) and transfected by calcium phosphate coprecipitation as described previously(Fischer et al., 1994). Transient transfections were carried out with 20µg of expression plasmid.

Recombinant protein was harvested by applying serum-free full medium to the transfected cells upon confluency (generally 48 hours post-transfection), after washing them twice with PBS (Ca²⁺ and Mg²⁺ free, Life Technologies). Conditioned medium was collected and cleared by centrifugation. Adherent cells were trypsinized, washed with PBS and the total cell number was determined by a CASY counter (Scharfe Systems, Germany) employing a 30µm capillary. Cell extracts were prepared by lysing the cells at a concentration of 5x10⁷ cells/ml lysis buffer, containing 20 mM Tris-HC1, pH7.5, 150mM NaCl, 1mM EDTA and 0.5 % Triton® X-100. After incubation for 30min at 4°C, lysates were cleared by centrifugation for 15 min at 10,000 x g at 4°C.

### 4. Western blotting.

Samples were reduced and denatured, resolved by SDS-PAGE on 4% stacking/8% or 10% separation gels, and visualized by Western blotting as described (Schlokat et al., 1996). Conditioned medium derived from FD11-CHO-rvWF transient transfections was concentrated 20x by speed-vac centrifugation prior to loading. Lysates were applied per slot on SDS-PAGE equivalent to 7.5x10 cells. For the detection of furin molecules, murine monoclonal antibody MON-148 (Alexis) directed against the catalytic domain of furin and alkaline phosphatase conjugated to anti-mouse IgG goat sera (Sigma) as the second antibody was used. Recombinant vWF was visualized employing rabbit anti-vWF antiserum (DAKO) and alkaline phosphatase conjugated to anti-rabbit IgG goat sera (Promega) as the second antibody.

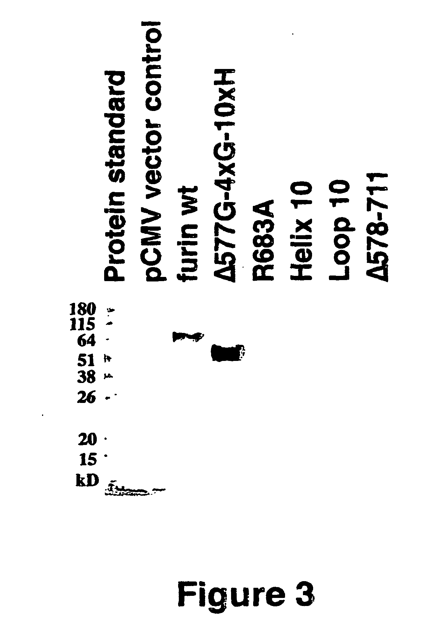

Fig. 3 shows the amount of shed furin in conditioned medium of transiently transfected FD11-CHO-rvWF cells. The conditioned medium was concentrated 20x and applied and denatured on 4% stacking/10% separation SDS-PAGE gel. The Western blot was visualized with MON-148 and AP-conjugated anti-mouse IgG antibody.

As a control, a pCMV vector, wild-type furin polypeptide and Δ577G-4xG-10xH were used. The furin construct Δ577G-4xG-10xH was prepared according to Preininger et al. (1999).

The figure clearly shows that the furin constructs according to the invention do not show any shedding, i.e. the secretion rate of the molecules into the medium is substantially reduced compared to rfurin having the wild-type sequence or furin lacking the transmembrane and cytoplasmic domains.

### 5. Analysis of in vitro furin activity in conditioned medium.

Functional activity of shed furin molecules was determined by fluorogenic substrate as described previously (Schlokat et al., 1996).

### 6. Evidence of intracellular rfurin activity.

FD11-CHO-rvWF cells stably expressing furin mutant R683A or wild-type furin were established by cotransfection using 20µg furin expression plasmid and 1µg selection plasmid pCMV-hyg mediating resistance to hygromycin B (Roche). Resistant clones were isolated two weeks after transfection and stabilized by subcloning under selective pressure. Three FD11-CHO-rvWF/R683A clones (clone 1, 2 and 3) differing in the amount of secreted rfurin and consequently showing variable degrees of rvWF precursor processing were selected.

Intracellular furin activity was demonstrated by correlating the degree of rvWF precursor processing and the presence of shed rfurin in FD11-CHO-rvWF/R683A conditioned media over a time period of 24 hours. As controls, FD11-CHO-rvWF/furin wt and FD11-CHO-rvWF cells were used. Cells were grown in 6-well dishes (one well/timepoint) until confluency, and washed two times with PBS before serum-free medium was applied for a time period of 4, 8, 16 and 24 hours. Conditioned medium was cleared by centrifugation and concentrated 20x for the detection of shed rfurin. Estimation of rvWF precursor processing was done by Western blot.

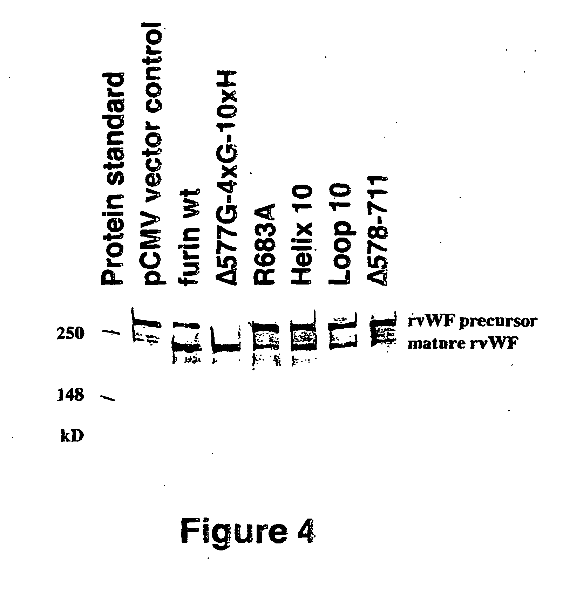

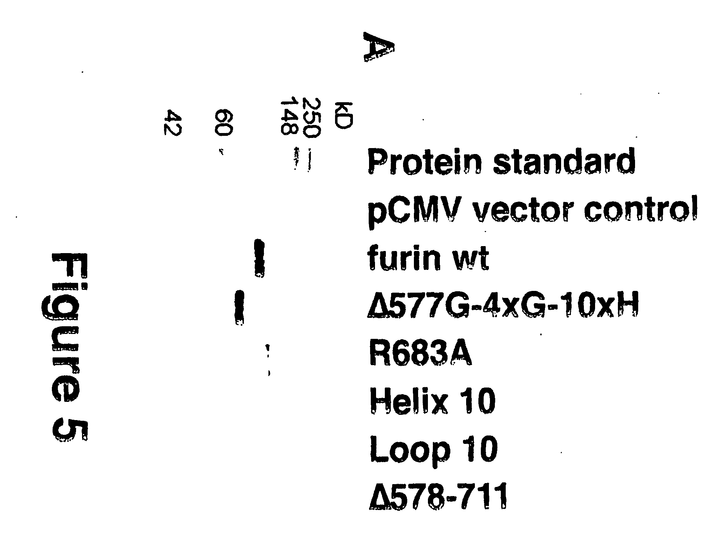

Fig. 4 shows the processing of rvWF precursor in transiently transfected FD11-CHO-rvWF cells. 100ng rvWF was applied per lane. Probes were reduced denatured and applied on 4% stacking/5% separating SDS-PAGE gel. The Western blot was developed with polyclonal rabbit-anti-vWF and AP-conjugated anti-rabbit IgG antibody. Although the cells were only transiently transfected, the R683A, Helix 10, Loop 10 and Δ578-711 furin constructs evidence proteolytic activity. The term transiently transfected reflects a genetically non-homogenous, mixed cell population. Depending on the transfection efficiency, only some of the cells are transfected.

Fig. 5 (comprising Figs. 5A-5C) shows furin expression in transiently transfected HEK293 cells:
Figure 5A shows shed furin in conditioned medium of transiently transfected HEK293 cells. 15µl of conditioned medium were applied per slot. Probes were reduced and denatured and applied on 4% stacking/10% separating SDS-PAGE. The Western blot was developed with MON-148 and AP-conjugated anti-mouse IgG antibody.
Figure 5B shows the measurement of intracellular rfurin in HEK293 lysates. 7.5x10e5 cell equivalents were applied per slot.
Figure 5C shows the results of an in vitro assay using conditioned medium and a fluorogenic substrate.

Fig. 5A shows that the amount of secreted furin polypeptides in the medium detectable by a specific antibody is highly reduced. This is confirmed by the in vitro activity measurements shown in Figure 5C. The data of Figure 5B show that the furin polypeptides are located intracellularly.

Fig. 6 shows the intracellular proteolytic activity of the furin construct R683A. The degree of rvWF precursor protein processing and the presence of shed rfurin in the conditioned medium is compared. The figure shows that significant proteolytic processing of vWF protein occurs even though no shed furin is detected in the medium. This indicates that this furin polypeptide is proteolytically active even though it is not secreted into the medium.

The upper lane is a vWF western blot, wherein 100ng rvWF is applied per lane. As a positive control, CHO-rvWf was used.

The lower lane is a furin western blot of conditioned medium. The material was concentrated 20x per lane. As a positive control, shed wild-type rvWF was used.

## Claims

1. A furin polypeptide comprising amino acids, said amino acids having a sequence which comprises a modification compared to the amino acid sequence of wild-type furin, wherein said modification is present between amino acids Ala 557 and Leu713 of wild-type furin, wherein said furin polypeptide has proteolytic activity, and wherein said furin polypeptide is not secreted into culture medium by host cells or which is secreted in reduced amounts compared to the secretion of wilde-type furin.

2. The furin polypeptide according to claim 1, wherein said modification results in the formation of a loop or alpha-helix structure between amino acids Ala 557 and Leu713 of wild-type furin.

3. The furin polypeptide according to claim 1, wherein said modification is between amino acids 577 and 713.

4. The furin polypeptide according to anyone of claims 1 to 3, wherein said modification is a substitution of from 5 to 30 amino acids.

5. The furin polypeptide according to claim 1, wherein said modification is a substitution of 10 or more amino acids that results in the formation of a helix or -loop structure within the mutant furin polypeptide.

6. The furin polypeptide according to anyone of claims 1 to 5, wherein said modification comprises adding or substituting amino acids selected from the group consisting of alanine (A), leucine (L), phenylalanine (F), tryptophan (W), methionine (M), histidine (H), glutamine (Q) and valine (V).

7. The furin polypeptide according to anyone of claims 1 to 5, wherein said modification comprises adding or substituting amino acids selected from the group consisting of serine (S), isoleucine (I), threonine (T), glutamic acid (E), aspartic acid (D), lysine (K), arginine(R), glycine (G), tyrosine (Y), cysteine (C), asparagine (N), glutamine (Q), proline (P) and hydroxyproline.

8. The furin polypeptide according to claim 1, wherein amino acids 578 to 711 are deleted.

9. The furin polypeptide according to claim 1, wherein the amino acids between amino acids 577 and 713 are replaced by amino acids comprising the sequence AEMWHQAMEV.

10. The furin polypeptide according to claim 1, wherein the amino acids between amino acids 577 and 713 are replaced by amino acids comprising the sequence GSPNSQPYDG.

11. The furin polypeptide according to claim 1, wherein said modification is at Arg683.

12. A recombinant DNA molecule encoding a furin polypeptide according to anyone of claims 1 to 11.

13. A recombinant expression vector comprising a DNA molecule according to claim 12 operably linked to a heterologous expression control sequence permitting expression of said furin polypeptide.

14. A host cell comprising a recombinant DNA expression vector according to claim 13.

15. The host cell according to claim 14, which additionally comprises a polynuceotide encoding at least one recombinantly expressed precursor polypeptide, wherein said polypeptide is a substrate for the encoded furin polypeptide.

16. A method for the production of a furin polypeptide according to anyone of claims 1 to 11, said method comprising:
(a) growing in a nutrient medium a host cell comprising an expression vector, said expression vector comprising, in order in the direction of transcription:
- a transcriptional regulatory region and a translational initiation region which is functional in said host cell,
- a DNA sequence encoding a mutant furin polypeptide according to anyone of claims 1 to 11, and
- translational and transcriptional termination regions functional in said host cell,
- wherein expression of said DNA sequence is regulated by said initiation and termination regions;
(b) measuring the secretion rate of furin polypeptides with proteolytic activity; and
(c) isolating furin polypeptides showing reduced secretion.

17. A pharmaceutical preparation comprising a furin polypeptide according to anyone of claims 1 to 11.

## Patentansprüche

1. Furin-Polypeptid, umfassend Aminosäuren, wobei die Aminosäuren eine Sequenz aufweisen, welche eine Modifikation, verglichen mit der Aminosäuresequenz von Wildtyp-Furin, umfasst, wobei die Modifikation zwischen den Aminosäuren Ala 557 und Leu 713 von Wildtyp-Furin vorliegt, wobei das Furin-Polypeptid proteolytische Aktivität aufweist, und wobei das Furin-Polypeptid nicht von Wirtszellen in das Kulturmedium sezemiert wird, oder welches in reduzierten Mengen, verglichen mit der Sekretion von Wildtyp-Furin, sezerniert wird.

2. Furin-Polypeptid nach Anspruch 1, wobei die Modifikation zur Bildung einer Schleifen- oder Alpha-Helix-Struktur zwischen den Aminosäuren Ala 557 und Leu 713 von Wildtyp-Furin führt.

3. Furin-Polypeptid nach Anspruch 1, wobei die Modifikation zwischen den Aminosäuren 577 und 713 liegt.

4. Furin-Polypeptid nach einem der Ansprüche 1 bis 3, wobei die Modifikation eine Substitution von 5 bis 30 Aminosäuren ist.

5. Furin-Polypetid nach Anspruch 1, wobei die Modifikation eine Substitution von 10 oder mehr Aminosäuren ist, welche zur Bildung einer Helix- oder Schleifen-Struktur innerhalb des mutierten Furin-Polypeptids führt.

6. Furin-Polypeptid nach einem der Ansprüche 1 bis 5, wobei die Modifikation das Hinzufügen oder Substituieren von Aminosäuren, ausgewählt aus der Gruppe, bestehend aus Alanin (A), Leucin (L), Phenylalanin (F), Tryptophan (W). Methionin (M), Histidin (H), Glutamin (Q) und Valin (V), umfasst.

7. Furin-Polypeptid nach einem der Ansprüche 1 bis 5, wobei die Modifikation das Hinzufügen oder Substituieren von Aminosäuren, ausgewählt aus der Gruppe, bestehend aus Serin (S), Isoleucin (I), Threonin (T), Glutaminsäure (E), Asparaginsäure (D), Lysin (K), Arginin (R), Glycin (G), Tyrosin (Y), Cystein (C), Asparagin (N), Glutamin (Q), Prolin (P) und Hydroxyprolin, umfasst.

8. Furin-Polypeptid nach Anspruch 1, wobei die Aminosäuren 578 bis 711 deletiert sind.

9. Furin-Polypeptid nach Anspruch 1, wobei die Aminosäuren zwischen den Aminosäuren 577 und 713 durch Aminosäuren ersetzt sind, welche die Sequenz AEMWHQAMEV umfassen.

10. Furin-Polypeptid nach Anspruch 1, wobei die Aminosäuren zwischen den Aminosäuren 577 und 713 durch Aminosäuren ersetzt sind, welche die Sequenz GSPNSQPYDG umfassen,

11. Furin-Polypeptid nach Anspruch 1, wobei die Modifikation bei Arg 683 vorliegt.

12. Rekombinantes DNA-Molekül, welches ein Furin-Polypeptid nach einem der Ansprüche 1 bis 11 codiert.

13. Rekombinanter Expressionsvektor, umfassend ein DNA-Molekül nach Anspruch 12, welches funktionell an eine heterologe Expressions-Kontroll-Sequenz gebunden ist, welcher die Expression des Furin-Polypeptids erlaubt.

14. Wirtszelle, umfassend einen rekombinanten DNA-Expressionsvektor nach Anspruch 13.

15. Wirtszelle nach Anspruch 14, welche zusätzlich ein Polynukleotid umfasst, welches mindestens ein rekombinant exprimiertes Vorläufer-Polypeptid codiert, wobei das Polypeptid ein Substrat für das codierte Furin-Polypeptid ist.

16. Verfahren zur Herstellung eines Furin-Polypeptids nach einem der Ansprüche 1 bis 11, wobei das Verfahren:
(a) das Züchten einer Wirtszelle in einem Nährmedium, umfassend einen Expressionsvektor, wobei der Expressionsvektor der Reihe nach in Transkriptionsrichtung umfasst:
- einen Transkriptions-Regulationsbereich und einen Translationslnitiationsbereich, welcher in der Wirtszelle funktionsfähig bzw. wirksam ist,
- eine DNA-Sequenz, welche ein mutiertes Furin-Polypeptid nach einem der Ansprüche 1 bis 11 codiert, und
- in der Wirtszelle funktionsfähige bzw. wirksame translationale und transkriptionale Terrninationsbereiche,
- wobei die Expression der DNA-Sequenz durch die Initiations- und Terminationsbereiche reguliert wird;
(b) das Messen der Sekretionsrate von Furin-Polypeptiden mit proteolytischer Aktivität; und
(c) das Isolieren von Furin-Polypeptiden, welche eine reduzierte Sekretion zeigten, umfasst.

17. Pharmazeutische Zubereitung, umfassend ein Furin-Polypeptid nach einem der Ansprüche 1 bis 11.

## Revendications

1. Polypeptide de furine comprenant des acides aminés, lesdits acides aminés ayant une séquence qui comprend une modification par rapport à la séquence d'acides aminés de la furine de type sauvage, dans lequel ladite modification est présente entre les acides aminés Ala 557 et Leu 713 de la furine de type sauvage, ledit polypeptide de furine a une activité protéolytique et ledit polypeptide de furine n'est pas sécrété dans le milieu de culture par des cellules hôtes ou est sécrété en quantités réduites en comparaison de la sécrétion de furine de type sauvage.

2. Polypeptide de furine selon la revendication 1, dans lequel ladite modification entraîne la formation d'une structure en boucle ou en hélice alpha entre les acides aminés Ala 557 et Leu 713 de la furine de type sauvage.

3. Polypeptide de furine selon la revendication 1, dans lequel ladite modification se trouve entre les acides aminés 577 et 713.

4. Polypeptide de furine selon l'une quelconque des revendications 1 à 3, dans lequel ladite modification est une substitution de 5 à 30 acides aminés.

5. Polypeptide de furine selon la revendication 1, dans lequel ladite modification est une substitution de 10 acides aminés ou plus qui entraîne la formation d'une structure en hélice ou en boucle dans le polypeptide de furine mutant.

6. Polypeptide de furine selon l'une quelconque des revendications 1 à 5, dans lequel ladite modification comprend l'addition ou la substitution d'acides aminés choisis dans le groupe constitué de l'alanine (A), de la leucine (L), de la phénylalanine (F), du tryptophane (W), de la méthionine (M), de l'histidine (H), de la glutamine (Q) et de la valine (V).

7. Polypeptide de furine selon l'une quelconque des revendications 1 à 5, dans lequel ladite modification comprend l'addition ou la substitution d'acides aminés choisis dans le groupe constitué de la sérine (S), de l'isoleucine (I), de la thréonine (T), de l'acide -glutamique (E), de l'acide aspartique (D), de la lysine (K), de l'arginine (R), de la glycine (G), de la tyrosine (T), de la cystéine (C), de l'asparagine (N), de la glutamine (Q), de la proline (P) et de l'hydroxyproline.

8. Polypeptide de furine selon la revendication 1, dans lequel les acides aminés 578 à 711 sont délétés.

9. Polypeptide de furine selon la revendication 1, dans lequel les acides aminés situés entre les acides aminés 577 et 713 sont remplacés par des acides aminés comprenant la séquence AEMWHQAMEV.

10. Polypeptide de furine selon la revendication 1, dans lequel les acides aminés situés entre les acides aminés 577 et 713 sont remplacés par des acides aminés comprenant la séquence GSPNSQPYDG.

11. Polypeptide de furine selon la revendication 1, dans lequel ladite modification est en position Arg 683.

12. Molécule d'ADN recombinant codant pour un polypeptide de furine selon l'une quelconque des revendications 1 à 11.

13. Vecteur d'expression recombinant comprenant une molécule d'ADN selon la revendication 12, liée de manière opérationnelle à une séquence de régulation d'expression hétérologue permettant l'expression dudit polypeptide de furine.

14. Cellule hôte comprenant un vecteur d'expression d'ADN recombinant selon la revendication 13.

15. Cellule hôte selon la revendication 14, qui comprend, en outre; un polynucléotide codant pour au moins un polypeptide précurseur exprimé de manière recombinante, dans laquelle ledit polypeptide est un substrat pour le polypeptide de furine codé.

16. Procédé pour la production d'un polypeptide de furine selon l'une quelconque des revendications 1 à 11, ledit procédé comprenant :
(a) la croissance dans un milieu nutritif d'une cellule hôte comprenant un vecteur d'expression, ledit vecteur d'expression comprenant dans l'ordre selon le sens de transcription :
- une région de régulation de la transcription et une région d'initiation de la traduction qui est fonctionnelle dans ladite cellule hôte.
- une séquence d'ADN codant pour un polypeptide de furine mutant, selon l'une quelconque des revendications 1 à 11, et
- des régions de terminaison de la traduction et de la transcription, fonctionnelles dans ladite cellule hôte,
- dans lequel l'expression de ladite séquence d'ADN est sous le contrôle desdites régions d'initiation et de terminaison;
(b) la mesure du taux de sécrétion de polypeptides de furine ayant une activité protéolytique; et
(c) l'isolement des polypeptides de furine présentant une sécrétion réduite.

17. Préparation pharmaceutique comprenant un polypeptide de furine selon l'une quelconque des revendications 1 à 11.


### SEQUENCE LISTING

<110> BAXTER Aktiengesellschaft
   Plaimauer, Barbara
   SCHLOKAT, Uwe
<120>
<130>
<150> 09/592480
   <151> 2000-06-09
<160> 16
<170> PatentIn Ver. 2.1
<210> 1
   <211> 5
   <212> PRT
   <213> Artificial Sequence
<220>
   <223> Description of Artificial Sequence:substitution region
<400> 1
   Glu Ala Met His Ala
    1               5
<210> 2
   <211> 5
   <212> PRT
   <213> Artificial Sequence
<220>
   <223> Description of Artificial Sequence:substitution region
<400> 2
   Ala Trp Phe Gln Trp
    1               5
<210> 3
   <211> 20
   <212> PRT
   <213> Artificial Sequence
<220>
   <223> Description of Artificial Sequence:substitution region
<400> 3
   Ala Gln Met Trp His Glu Ala Met Glu Phe Trp Ala Met Gln Phe Glu
   1 5 10 15
   Ala Met His Ala
   20
<210> 4
   <211> 10
   <212> PRT
   <213> Artificial Sequence
<220>
   <223> Description of Artificial Sequence:substitution region
<400> 4
   Ala Glu Met Trp His Gln Ala Met Glu Val
   1 5 10
<210> 5
   <211> 10
   <212> PRT
   <213> Artificial Sequence
<220>
   <223> Description of Artificial Sequence:substitution region
<400> 5
   Gly Ser Pro Asn Ser Gln Pro Tyr Asp Gly
   1 5 10
<210> 6
   <211> 22
   <212> DNA
   <213> Artificial Sequence
<220>
   <223> Description of Artificial Sequence:primer
<400> 6
   gggggatccc tctggcgagt gg 22
<210> 7
   <211> 21
   <212> DNA
   <213> Artificial Sequence
<220>
   <223> Description of Artificial Sequence:primer
<400> 7
   cggggactct gcgctgctct g 21
<210> 8
   <211> 21
   <212> DNA
   <213> Artificial Sequence
<220>
   <223> Description of Artificial Sequence:primer
<400> 8
   cagagcagcg cagagtcccc g 21
<210> 9
   <211> 21
   <212> DNA
   <213> Artificial Sequence
<220>
   <223> Description of Artificial Sequence:primer
<400> 9
   gggggatccc cgcggcctag g 21
<210> 10
   <211> 45
   <212> DNA
   <213> Artificial Sequence
<220>
   <223> Description of Artificial Sequence:primer
<400> 10
   caggccatgg aggtgcacct gcctgaggtg gtggccggcc tcagc 45
<210> 11
   <211> 45
   <212> DNA
   <213> Artificial Sequence
<220>
   <223> Description of Artificial Sequence:primer
<400> 11
   gtgccacatc tcggccccct caggggcggt gccatagagt acgag 45
<210> 12
   <211> 45
   <212> DNA
   <213> Artificial Sequence
<220>
   <223> Description of Artificial Sequence:primer
<400> 12
   cagccctacg acggccacct gcctgaggtg gtggccggcc tcagc 45
<210> 13
   <211> 45
   <212> DNA
   <213> Artificial Sequence
<220>
   <223> Description of Artificial Sequence:primer
<400> 13
   gctgttgggg ctgcccccct caggggcggt gccatagagt acgag 45
<210> 14
   <211> 21
   <212> DNA
   <213> Artificial Sequence
<220>
   <223> Description of Artificial Sequence:primer
<400> 14
   cacctgcctg aggtggtggc c 21
<210> 15
   <211> 21
   <212> DNA
   <213> Artificial Sequence
<220>
   <223> Description of Artificial Sequence:primer
<400> 15
   cccctcaggg gcggtgccat a 21
<210> 16
   <211> 794
   <212> PRT
   <213> human
<400> 16
   Met Glu Leu Arg Pro Trp Leu Leu Trp Val Val Ala Ala Thr Gly Thr
    1               5                  10                  15
   Leu Val Leu Leu Ala Ala Asp Ala Gln Gly Gln Lys Val Phe Thr Asn
               20                  25                  30
   Thr Trp Ala Val Arg Ile Pro Gly Gly Pro Ala Val Ala Asn Ser Val
           35                  40                  45
   Ala Arg Lys His Gly Phe Leu Asn Leu Gly Gln Ile Phe Gly Asp Tyr
       50                  55                  60
   Tyr His Phe Trp His Arg Gly Val Thr Lys Arg Ser Leu Ser Pro His
   65                  70                  75                  80
   Arg Pro Arg His Ser Arg Leu Gln Arg Glu Pro Gln Val Gln Trp Leu
                   85                  90                  95
   Glu Gln Gln Val Ala Lys Arg Arg Thr Lys Arg Asp Val Tyr Gln Glu
               100                 105                 110
   Pro Thr Asp Pro Lys Phe Pro Gln Gln Trp Tyr Leu Ser Gly Val Thr
           115                 120                 125
   Gln Arg Asp Leu Asn Val Lys Ala Ala Trp Ala Gln Gly Tyr Thr Gly
   130 135 140
   His Gly Ile Val Val Ser Ile Leu Asp Asp Gly Ile Glu Lys Asn His
   145 150 155 160
   Pro Asp Leu Ala Gly Asn Tyr Asp Pro Gly Ala Ser Phe Asp Val Asn
   165 170 175
   Asp Gln Asp Pro Asp Pro Gln Pro Arg Tyr Thr Gln Met Asn Asp Asn
   180 185 190
   Arg His Gly Thr Arg Cys Ala Gly Glu Val Ala Ala Val Ala Asn Asn
   195 200 205
   Gly Val Cys Gly Val Gly Val Ala Tyr Asn Ala Arg Ile Gly Gly Val
   210 215 220
   Arg Met Leu Asp Gly Glu Val Thr Asp Ala Val Glu Ala Arg Ser Leu
   225 230 235 240
   Gly Leu Asn Pro Asn His Ile His Ile Tyr Ser Ala Ser Trp Gly Pro
   245 250 255
   Glu Asp Asp Gly Lys Thr Val Asp Gly Pro Ala Arg Leu Ala Glu Glu
   260 265 270
   Ala Phe Phe Arg Gly Val Ser Gln Gly Arg Gly Gly Leu Gly Ser Ile
   275 280 285
   Phe Val Trp Ala Ser Gly Asn Gly Gly Arg Glu His Asp Ser Cys Asn
   290 295 300
   Cys Asp Gly Tyr Thr Asn Ser Ile Tyr Thr Leu Ser Ile Ser Ser Ala
   305 310 315 320
   Thr Gln Phe Gly Asn Val Pro Trp Tyr Ser Glu Ala Cys Ser Ser Thr
   325 330 335
   Leu Ala Thr Thr Tyr Ser Ser Gly Asn Gln Asn Glu Lys Gln Ile Val
   340 345 350
   Thr Thr Asp Leu Arg Gln Lys Cys Thr Glu Ser His Thr Gly Thr Ser
   355 360 365
   Ala Ser Ala Pro Leu Ala Ala Gly Ile Ile Ala Leu Thr Leu Glu Ala
   370 375 380
   Asn Lys Asn Leu Thr Trp Arg Asp Met Gln His Leu Val Val Gln Thr
   385 390 395 400
   Ser Lys Pro Ala His Leu Asn Ala Asn Asp Trp Ala Thr Asn Gly Val
   405 410 415
   Gly Arg Lys Val Ser His Ser Tyr Gly Tyr Gly Leu Leu Asp Ala Gly
   420 425 430
   Ala Met Val Ala Leu Ala Gln Asn Trp Thr Thr Val Ala Pro Gln Arg
   435 440 445
   Lys Cys Ile Ile Asp Ile Leu Thr Glu Pro Lys Asp Ile Gly Lys Arg
   450 455 460
   Leu Glu Val Arg Lys Thr Val Thr Ala Cys Leu Gly Glu Pro Asn His
   465 470 475 480
   Ile Thr Arg Leu Glu His Ala Gln Ala Arg Leu Thr Leu Ser Tyr Asn
   485 490 495
   Arg Arg Gly Asp Leu Ala Ile His Leu Val Ser Pro Met Gly Thr Arg
   500 505 510
   Ser Thr Leu Leu Ala Ala Arg Pro His Asp Tyr Ser Ala Asp Gly Phe
   515 520 525
   Asn Asp Trp Ala Phe Met Thr Thr His Ser Trp Asp Glu Asp Pro Ser
   530 535 540
   Gly Glu Trp Val Leu Glu Ile Glu Asn Thr Ser Glu Ala Asn Asn Tyr
   545 550 555 560
   Gly Thr Leu Thr Lys Phe Thr Leu Val Leu Tyr Gly Thr Ala Pro Glu
   565 570 575
   Gly Leu Pro Val Pro Pro Glu Ser Ser Gly Cys Lys Thr Leu Thr Ser
   580 585 590
   Ser Gln Ala Cys Val Val Cys Glu Glu Gly Phe Ser Leu His Gln Lys
   595 600 605
   Ser Cys Val Gln His Cys Pro Pro Gly Phe Ala Pro Gln Val Leu Asp
   610 615 620
   Thr His Tyr Ser Thr Glu Asn Asp Val Glu Thr Ile Arg Ala Ser Val
   625 630 635 640
   Cys Ala Pro Cys His Ala Ser Cys Ala Thr Cys Gln Gly Pro Ala Leu
                   645                 650                 655
   Thr Asp Cys Leu Ser Cys Pro Ser His Ala Ser Leu Asp Pro Val Glu
               660                 665                 670
   Gln Thr Cys Ser Arg Gln Ser Gln Ser Ser Arg Glu Ser Pro Pro Gln
           675                 680                 685
   Gln Gln Pro Pro Arg Leu Pro Pro Glu Val Glu Ala Gly Gln Arg Leu
       690                 695                 700
   Arg Ala Gly Leu Leu Pro Ser His Leu Pro Glu Val Val Ala Gly Leu
   705                 710                 715                 720
   Ser Cys Ala Phe Ile Val Leu Val Phe Val Thr Val Phe Leu Val Leu
                   725                 730                 735
   Gln Leu Arg Ser Gly Phe Ser Phe Arg Gly Val Lys Val Tyr Thr Met
               740                 745                 750
   Asp Arg Gly Leu Ile Ser Tyr Lys Gly Leu Pro Pro Glu Ala Trp Gln
           755                 760                 765
   Glu Glu Cys Pro Ser Asp Ser Glu Glu Asp Glu Gly Arg Gly Glu Arg
       770                 775                 780
   Thr Ala Phe Ile Lys Asp Gln Ser Ala Leu
   785                 790